Europäisches Patentamt

European Patent Office

Office européen des brevets

(19)

(11) Veröffentlichungsnummer : **0 093 266**
**B1**

# (12) EUROPÄISCHE PATENTSCHRIFT

(45) Veröffentlichungstag der Patentschrift :
**09.10.85**

(21) Anmeldenummer : **83103108.3**

(22) Anmeldetag : **29.03.83**

(51) Int. Cl.⁴ : **C 07 J 19/00**

(54) Verfahren zur Herstellung von beta-Methyldigoxin.

(30) Priorität : 30.04.82 DE 3216236

(43) Veröffentlichungstag der Anmeldung :
09.11.83 Patentblatt 83/45

(45) Bekanntmachung des Hinweises auf die Patenterteilung : 09.10.85 Patentblatt 85/41

(84) Benannte Vertragsstaaten :
AT BE CH DE FR GB IT LI LU NL SE

(56) Entgegenhaltungen :
FR-A- 2 191 902
FR-A- 2 362 160

(73) Patentinhaber : CHEMIE LINZ AKTIENGESELL-
SCHAFT
St. Peter-Strasse 25
A-4020 Linz (AT)
BE CH FR GB IT LI LU NL SE AT
Lentia Gesellschaft mit beschränkter Haftung
Arabellastrasse 4 Postfach 81 05 08
D-8000 München 81 (DE)
DE

(72) Erfinder : Franzmair, Rudolf, Dr.
Franz-Xaver-Müllerweg 9
A-4033 Ebelsberg (AT)
Erfinder : Schneider, Herwig
Unionstrasse 147
A-4020 Linz (AT)
Erfinder : Becker, Josef
Nietzschestrasse 4
A-4020 Linz (AT)

# 0 093 266

**Beschreibung**

Die Erfindung betrifft ein Verfahren zur Herstellung von 4'''-O-Methyldigoxin (β-Methyldigoxin) durch partielle Methylierung von Digoxin.

Die Herstellung von Digoxinmonoalkyläthern durch Monomethylverätherung von Digoxin ist aus der Patentliteratur bekannt. Nach dem Verfahren der NL-A-6 813 409 ist die partielle Monoalkylierung der 3'''- oder 4'''-ständigen Hydroxygruppe an der endständigen Digitoxose mit den üblichen Methylierungsmitteln, wie Diazomethan oder Dimethylsulfat, unter den an sich bekannten Verfahrensbedingungen durchführbar. Bei der Methylierung mit Dimethylsulfat entstehen aber in Mengen von 20 bis 30 Gew.% Digoxinpolyäther als Nebenprodukte, die nicht mehr zu Digoxin oder Monomethyldigoxin entmethyliert werden können. Diese Nebenprodukte müssen auf chromatographischem Weg abgetrennt werden und bedeuten einen erheblichen Verlust an teurem Ausgangsmaterial.

Die Verwendung des giftigen und explosiven Diazomethans zur Monoalkylierung von Digoxin ist für ein großtechnisches Herstellungsverfahren nicht geeignet.

Nach einem verbesserten Verfahren, wie es in der DE-B-1 961 034 beschrieben ist, kann die Anwendung von Diazomethan vermieden werden und die Selektivität der Monoalkylierungsreaktion begünstigt werden, wenn man die an sich bekannte Umsetzung von Digoxin mit Dimethylsulfat in Gegenwart von Bariumhydroxid und einer basischen Aluminiumverbindung ausführt. Nach diesem Verfahren ist eine 45 %ige Ausbeute an β-Methyldigoxin zu erreichen, wenn man die Umsetzung in einer Mischung aus Dimethylformamid und Toluol bei Raumtemperatur vornimmt und dem Reaktionsgemisch Aluminiumoxid zusetzt. Zur Reinigung des rohen β-Methyldigoxin, welches neben nicht umgesetztem Digoxin Beimengungen an α-Methyl- und Dimethyl-digoxin enthält, wird das Reaktionsgemisch unter Zusatz von Pyridin im Vakuum eingeengt und der Rückstand nach der Methode der multiplikativen Verteilung zwischen zwei Lösungsmittelphasen von nicht umgesetztem Digoxin befreit. Das in der wässerigen Phase befindliche Digoxin wird mit Chloroform extrahiert und einem weiteren Methylierungsvorgang zugeführt.

Aus der DE-A-2 233 147 ist es bekannt, β-Methyldigoxin als Ausgangsmaterial zur Synthese von 20,22-Dihydro-4'''-O-methyldigoxin herzustellen, indem man Digoxin in Anwesenheit von Strontiumhydroxid und Aluminiumoxid bei Raumtemperatur mit Dimethylsulfat umsetzt und das Rohprodukt durch multiplikative Verteilung, Chloroformextraktion und Umkristallisieren aus Aceton reinigt. Im einzigen Beispiel dieser Offenlegungschrift, das die Herstellung von β-Methyldigoxin betrifft, wird mit einem molaren Verhältnis von Dimethylsulfat zu Digoxin von 19 : 1 gearbeitet. Dieses Verfahren hat den Nachteil, daß zur Methylierung ein großer Überschuß an Dimethylsulfat benötigt wird, der bei der Aufarbeitung des Rohproduktes wieder entfernt werden muß.

Schließlich ist noch in der DE-B-2 734 401 die Herstellung von β-Methyldigoxin durch Umsetzung von Digoxin mit Methylestern organischer oder anorganischer Schwefelsäuren in einer Inertgasatmosphäre und die Reinigung des Methylierungsprodukts durch Säulenchromatographie an $SiO_2$ beschrieben.

Nach dem zuletzt genannten Verfahren wird die Umsetzung bei Verwendung von Dimethylsulfat als Methylierungsmittel in einem Gemisch aus trockenem Dimethylformamid und Dioxan in Gegenwart von Strontiumhydroxid und Aluminiumoxid bei Raumtemperatur ausgeführt.

Es wurde nunmehr gefunden, daß bei der Monomethylierung von Digoxin in der 16-Stellung der endständigen Digitoxose hinsichtlich Selektivität und Ausbeute der Reaktion überraschenderweise bessere Ergebnisse als die in der Patentliteratur angegebenen erzielt werden, wenn man die Umsetzung bei tieferen Temperaturen als der Raumtemperatur in Anwesenheit einer wasserhaltigen basischen Strontiumverbindung und gegebenenfalls eines inerten anorganischen Adsorbens durchführt, das ebenfalls Wasser enthalten kann.

Gegenstand der Erfindung ist demnach ein Verfahren zur Herstellung von β-Methyldigoxin durch selektive Monomethylierung von Digoxin mit Dimethylsulfat in einem Dimethylformamid-Toluol-Gemisch als Lösungsmittel in Anwesenheit einer basischen Strontiumverbindung und gegebenenfalls eines inerten anorganischen Adsorbens sowie Reinigung des Methylierungsproduktes durch Säulenchromatographie an $SiO_2$ mit einem Gemisch aus chloriertem Kohlenwasserstoff und einem niederen aliphatischen Alkohol, welches dadurch gekennzeichnet ist, daß pro Mol Digoxin als basische Strontiumverbindung 1 bis 3 Mol Strontiumhydroxidoctahydrat und als gegebenenfalls vorhandenes inertes anorganisches Adsorbens 1 bis 3 Mol eines Oxids, Silikats oder Phosphats von Elementen der 2., 3., 4. oder 5. Haupt- oder Nebengruppe des Periodensystems nach Mendelejeff mit einem Wassergehalt bis zu 20 Gew.% eingesetzt werden und die Umsetzung bei Temperaturen von − 15 °C bis 15 °C durchgeführt wird.

Gemäß einer bevorzugten Ausführungsform der vorliegenden Erfindung erfolgt die Methylierung bei einer Temperatur von − 10 bis 10, vorzugsweise − 5 bis 5 °C.

Im allgemeinen wird das erfindungsgemäße Verfahren so durchgeführt, daß man eine Lösung von reinem Digoxin in einem Dimethylformamid-Toluol Gemisch vorlegt und diese dann mit Strontiumhydroxydoctahydrat und gegebenenfalls dem inerten anorganischen Adsorbens versetzt. Nach dem Durchmischen und Einstellen der gewünschten Reaktionstemperatur wird Dimethylsulfat in Toluol gelöst zugetropft.

2

Verwendet man zum Regulieren des pH-Wertes und zur Begünstigung des selektiven Verlaufs der Methylierung erfindungsgemäß Strontiumhydroxidoctahydrat, so ist es unerwarteterweise zur Erzielung guter Ausbeuten und eines selektiven Verlaufs der Monomethylierung nicht unbedingt notwendig, dem Reaktionsgemisch ein inertes wasserhaltiges anorganisches Adsorbens zuzusetzen.

Wird die Reaktion aber zusätzlich in Gegenwart eines anorganischen Adsorbens ausgeführt, so sind bevorzugte anorganische Adsorbentien die Oxide von Elementen der 2., 3. und 4. Hauptgruppe des Periodensystems nach Mendelejeff mit einem Wassergehalt von 10-20 Gew.%, wobei wiederum Aluminiumoxid, Kieselgel, Titandioxid, Kaolin und Talkum besonders bevorzugt sind.

Das molare Verhältnis von Digoxin zu Strontiumhydroxidoctahydrat zu Dimethylsulfat beträgt im allgemeinen 1 : 1 bis 3 : 1 bis 5. Innerhalb dieser Grenzen werden in bevorzugter und sparsamer Weise 2 Mol Strontiumhydroxydoctahydrat pro Mol Digoxin eingesetzt. Führt man das erfindungsgemäße Verfahren in Anwesenheit eines anorganischen Adsorbens aus, so empfiehlt es sich, pro Mol Digoxin 1 bis 3 Mol des Adsorbens, vorzugsweise 3 Mol einzusetzen. Die Menge an Dimethylsulfat die pro Mol Digoxin eingesetzt wird, beträgt vorzugsweise 2-3 Mol.

Das auf übliche Weise vorgereinigte Methylierungsprodukt wird zur säulenchromatographischen Trennung auf Kieselgel aufgegeben und mit einem Gemisch aus einem halogeniertem Kohlenwasserstoff und einem niederen aliphatischen Alkohol, vorzugsweise einem Gemisch von Methylenchlorid zu Methanol von 9 : 1 eluiert und fraktioniert. Die Fraktionen, welche reines β-Methyldigoxin enthalten, werden vereinigt und im Vakuum vom Elutionsmittel befreit. Das auf diese Weise isolierte β-Methyldigoxin kann anschließend beispielsweise aus Aceton umkristallisiert werden.

Das reine nach dem erfindungsgemäßen Verfahren hergestellte β-Methyldigoxin wird durch Dünnschichtchromatographie, Schmelzpunkt und IR-Spektren identifiziert.

Die Erfindung wird durch die nachfolgenden Beispiele näher erläutert.

Die in den Beispielen angegebenen Ausbeuten und Selektivitäten der Reaktion werden nach folgenden Gleichungen bestimmt :

$$\text{Ausbeute (\%)} = \frac{\text{gebildetes } \beta\text{-Methyldigoxin in Mol}}{\text{eingesetztes Digoxin in Mol}} \times 100$$

$$\text{Selektivität (\%)} = \frac{\text{gebildetes } \beta\text{-Methyldigoxin in Mol}}{\text{umgesetztes Digoxin in Mol}} \times 100$$

Beispiel 1

2,34 g (3 mmol) Digoxin werden in 30 ml Dimethylformamid warm gelöst, mit 30 ml Toluol versetzt und nach Zugabe von 1,59 g (6 mmol) Strontiumhydroxidoctahydrat und 1,13 g (9 mmol) Kieselgel mit einem Wassergehalt von 10 Gew.% wird das Gemisch eine halbe Stunde bei 0 °C gerührt.

Anschließend wird eine Lösung von 1,14 g (9 mmol) Dimethylsulfat in 15 ml Toluol innerhalb von einer halben Stunde zugetropft und das Reaktionsgemisch 20 Stunden bei 0 °C gerührt. Danach wird mit 150 ml Chloroform verdünnt, mit 3 ml Wasser kräftig ausgerührt, über Kieselgel filtriert, mit 75 ml Chloroform nachgewaschen und das Filtrat nach Zugabe von 5 ml Pyridin im Vakuum eingedampft. Der Trockenrückstand wird in 150 ml Chloroform aufgenommen und 3 mal mit je 50 ml Wasser gewaschen. Das vereinigte Waschwasser wird mit 50 ml Chloroform extrahiert, die Chloroformphasen über Natriumsulfat getrocknet und eingedampft. Der Rückstand wird an einer Kieselgelsäule adsorbiert und mit Methylenchlorid : Methanol 9 : 1 eluiert. Nach dem Eindampfen der gesammelten Fraktionen erhält man 200 mg (8,24 % d. Th.) Dimethyldigoxin, 200 mg (8,55 % d. Th.) Digoxin und 1,849 g (77,53 % d. Th.) β-Methyldigoxin, welches nach dem Umkristallisieren aus Aceton 1,801 g an reinem β-Methyldigoxin ergibt. Die Ausbeute beträgt 75,5 %, die Selektivität der Reaktion 82,57 %.

Beispiele 2, 3, 4 und 5

In den nachfolgenden Beispielen wird die Monomethylierung von Digoxin bei gleicher Ansatzgröße und gleicher Arbeitsweise wie in Beispiel 1, jedoch bei verschiedenen Reaktionstemperaturen ausgeführt. Reaktionstemperaturen, Ausbeuten und Selektivitäten der Reaktion sind in Tabelle I angegeben.

Tabelle I

| Beispiel | Reaktionstemperatur | Ausbeute | Selektivität |
|---|---|---|---|
| 2 | -5 °C | 73,08 % | 83,68 % |
| 3 | 5 °C | 74,34 % | 80,30 % |
| 4 | 10 °C | 71,28 % | 75,35 % |
| 5 | 15 °C | 63,65 % | 84,48 % |

**0 093 266**

### Beispiel 6

2,34 g (3 mmol) Digoxin werden in 30 ml Dimethylformamid warm gelöst, mit 30 ml Toluol versetzt und nach Zugabe von 2,39 g (9 mmol) Strontiumhydroxidoctahydrat wird das Gemisch eine halbe Stunde bei − 10 °C gerührt.

Anschließend wird eine Lösung von 1,14 g (9 mmol) Dimethylsulfat in 15 ml Toluol innerhalb von einer halben Stunde zugetropft und das Reaktionsgemisch 20 Stunden lang bei − 10 °C gerührt. Die Aufarbeitung erfolgt wie in Beispiel 1.

Nach dem Eindampfen der gesammelten Fraktionen erhält man 174 mg (7,4 % d. Th.) Dimethyldigoxin, 267 mg (11,4 % d. Th.) Digoxin und 1,836 g (78,29 % d. Th.) β-Methyldigoxin, woraus man nach dem Umkristallisieren aus Aceton 1,69 g reines β-Methyldigoxin gewinnt. Die Ausbeute beträgt 72,06 %, die Selektivität der Reaktion 79,98 %.

### Beispiel 7

Bei ansonst gleicher Ansatzgröße und gleichen Reaktionsbedingungen wie in Beispiel 6 angegeben, werden 1,59 g (6 mmol) Strontiumhydroxidoctahydrat zugesetzt.

Man erhält so nach dem Eindampfen der gesammelten Fraktionen und durch Umkristallisieren aus Aceton 1,76 g reines β-Methyldigoxin, was einer Ausbeute von 73,79 % d. Th. entspricht.

### Beispiel 8

Bei ansonst gleichen Mengenverhältnissen und gleicher Arbeitsweise wie in Beispiel 6, werden 1,59 g (6 mmol) Strontiumhydroxidoctahydrat zugegeben und die Umsetzung bei 0 °C ausgeführt.

Man erhält so nach dem Eindampfen der gesammelten Fraktionen und Umkristallisieren aus Aceton 1,82 g reines β-Methyldigoxin, was einer Ausbeute von 76,31 % d. Th. entspricht.

### Beispiel 9

Bei gleicher Arbeitsweise wie in Beispiel 6 wird eine Lösung von 2,34 g (3 mmol) Digoxin mit 797 mg (3 mmol) Strontiumhydroxidoctahydrat versetzt und mit 757 g (6 mmol) Dimethylsulfat bei 0 °C methyliert. Nach dem Eindampfen der gesammelten Fraktionen erhält man 119 mg (4,90 % d. Th.) Dimethyldigoxin, 403 mg (17,16 %) Digoxin und 1,732 g (72,62 % d. Th.) β-Methyldigoxin, woraus man nach dem Umkristallisieren an Aceton 1,59 g reines β-Methyldigoxin gewinnt. Die Ausbeute beträgt 66,67 %, die Selektivität der Reaktion 80,48 %.

### Beispiel 10

2,34 g Digoxin werden in 30 ml Dimethylformamid warm gelöst, mit 30 ml Toluol versetzt und nach Zugabe von 1,59 g (6 mmol) Strontiumhydroxidoctahydrat und 0,92 g (9 mmol) Aluminiumoxid mit einem Wassergehalt von 20 Gew.% wird das Gemisch eine halbe Stunde bei 0 °C gerührt. Anschließend wird eine Lösung von 1,14 g (9 mmol) Dimethylsulfat in 15 ml Toluol innerhalb von einer halben Stunde zugetropft und das Reaktionsgemisch 20 Stunden bei 0 °C gerührt. Die Aufarbeitung erfolgt wie in Beispiel 1.

Nach dem Eindampfen der gesammelten Fraktionen und Umkristallisieren aus Aceton erhält man 1,78 g reines β-Methyldigoxin, was einer Ausbeute von 74,63 % d. Th. entspricht.

### Beispiel 11

2,34 g (3 mmol) Digoxin werden in 30 ml Dimethylformamid warm gelöst, mit 30 ml Toluol versetzt und nach Zugabe von 1,59 g (6 mmol) Strontiumhydroxidoctahydrat und 719 mg (9 mmol) Titandioxid wird das Gemisch eine halbe Stunde bei 0 °C gerührt.

Anschließend wird die Lösung von 1,14 g (9 mmol) Dimethylsulfat in 15 ml Toluol innerhalb von einer halben Stunde zugetropft und das Reaktionsgemisch 20 Stunden lang bei 0 °C gerührt. Die Aufarbeitung erfolgt wie in Beispiel 1.

Nach dem Eindampfen der gesammelten Fraktionen erhält man 402 mg (16,6 % d. Th.) Dimethyldigoxin, 72 mg (3,07 % d. Th.) Digoxin und 1,727 g (72,41 % d. Th.) β-Methyldigoxin, woraus man nach dem Umkristallisieren aus Aceton 1,585 g reines β-Methyldigoxin erhält. Die Ausbeute beträgt 66,46 %, die Selektivität der Reaktion 68,56 %.

### Beispiel 12

Bei ansonst gleicher Ansatzgröße und gleichen Reaktionsbedingungen wie in Beispiel 11, werden als anorganisches Adsorbens 1,55 g (9 mmol) Calciumhydrogenphosphat-Dihydrat zugesetzt.

Man erhält so nach dem Eindampfen der gesammelten Fraktionen 367 mg (15,12 % d. Th.)

4

Dimethyldigoxin, 77 mg (3,29 % d. Th.) Digoxin und 1,636 g (68,6 % d. Th.) β-Methyldigoxin, woraus man nach dem Umkristallisieren von Aceton 1,425 g reines β-Methyldigoxin erhält. Die Ausbeute beträgt 59,75 %, die Selektivität der Reaktion 61,78 %.

Beispiel 13

Bei ansonst gleicher Ansatzgröße und gleichen Reaktionsbedingungen wie in Beispiel 11, werden als anorganisches Adsorbens 1,46 g (9 mmol) Kaolin zugesetzt.

Man erhält so nach dem Eindampfen der gesammelten Fraktionen 278 mg (11,44 % d. Th.) Dimethyldigoxin, 105 mg (4,48 % d. Th.) Digoxin und 1,733 g (72,66 % d. Th.) β-Methyldigoxin, woraus man nach dem Umkristallisieren aus Aceton 1,62 g reines β-Methyldigoxin gewinnt. Die Ausbeute beträgt 67,93 %, die Selektivität der Reaktion 71,11 %.

Beispiel 14

Bei ansonst gleicher Ansatzgröße und gleichen Reaktionsbedingungen wie in Beispiel 11, werden als anorganisches Adsorbens 1,5 g Talkum zugegeben.

Man erhält nach dem Eindampfen der gesammelten Fraktionen 356 mg (14,67 % d. Th.) Dimethyldigoxin, 99 mg (4,23 % d. Th.) Digoxin und 1,804 g (75,64 % d. Th.) β-Methyldigoxin, woraus man nach dem Umkristallisieren aus Aceton 1,694 g reines β-Methyldigoxin gewinnt. Die Ausbeute beträgt 71,03 %, die Selektivität der Reaktion 75,49 %.

## Patentansprüche

1. Verfahren zur Herstellung von β-Methyldigoxin durch selektive Monomethylierung von Digoxin mit Dimethylsulfat in einem Dimethylformamid-Toluol-Gemisch als Lösungsmittel in Anwesenheit einer basischen Strontiumverbindung und gegebenenfalls eines inerten anorganischen Adsorbens sowie Reinigung des Methylierungsproduktes durch Säulenchromatographie an $SiO_2$ mit einem Gemisch aus chloriertem Kohlenwasserstoff und einem niederen aliphatischen Alkohol, dadurch gekennzeichnet, daß pro Mol Digoxin als basische Strontiumverbindung 1 bis 3 Mol Strontiumhydroxidoctahydrat und als gegebenenfalls vorhandenes inertes anorganisches Adsorbens 1 bis 3 Mol eines Oxids, Silikats oder Phosphats von Elementen der 2., 3., 4. oder 5. Haupt- oder Nebengruppe des Periodensystems nach Mendelejeff mit einem Wassergehalt bis zu 20 Gew.% eingesetzt werden und die Umsetzung bei Temperaturen von − 15° bis 15 °C durchgeführt wird.

2. Verfahren nach Anspruch 1, dadurch gekennzeichnet, daß die Umsetzung bei Temperaturen von − 5 °C bis 5 °C durchgeführt wird.

3. Verfahren nach den Ansprüchen 1 und 2, dadurch gekennzeichnet, daß pro Mol Digoxin 2 Mol Strontiumhydroxidoctahydrat eingesetzt werden.

4. Verfahren nach den Ansprüchen 1 bis 3, dadurch gekennzeichnet, daß pro Mol Digoxin 3 Mol des inerten anorganischen Adsorbens eingesetzt werden.

5. Verfahren nach den Ansprüchen 1 bis 4, dadurch gekennzeichnet, daß als inertes anorganisches Adsorbens Oxide von Elementen der 2., 3. und 4. Hauptgruppe des Periodensystems nach Mendelejeff mit einem Wassergehalt von 10 bis 20 Gew.% eingesetzt werden.

6. Verfahren nach den Ansprüchen 1 bis 3, dadurch gekennzeichnet, daß die Monomethylierung von Digoxin in Anwesenheit von Strontiumhydroxidoctahydrat ohne Zusatz eines inerten anorganischen Adsorbens durchgeführt wird.

## Claims

1. A process for the preparation of β-methyldigoxin by the selective monomethylation of digoxin with dimethyl sulphate in a dimethylformamide/toluene mixture as the solvent, in the presence of a basic strontium compound and, if appropriate, of an inert inorganic adsorbent, and purification of the methylation product by column chromatography on $SiO_2$ with a mixture of chlorohydrocarbon and a lower aliphatic alcohol, characterised in that, per mole of digoxin, 1 to 3 moles of strontium hydroxide octahydrate are used as the basic strontium compound, and 1 to 3 moles of an oxide, silicate or phosphate of an element of the 2nd, 3rd, 4th or 5th main group or subgroup of Mendeleev's periodic table, with a water content of up to 20 % by weight, are used as the inert organic adsorbent which may be present, and the reaction is carried out at temperatures of − 15 °C to 15 °C.

2. The process as claimed in Claim 1, characterised in that the reaction is carried out at temperatures of − 5 °C to 5 °C.

3. The process as claimed in Claims 1 and 2, characterised in that 2 moles of strontium hydroxide octahydrate are used per mole of digoxin.

4. The process as claimed in Claims 1 to 3, characterised in that 3 moles of the inert inorganic adsorbent are used per mole of digoxin.

5. The process as claimed in Claims 1 to 4, characterised in that oxides of elements of the 2nd, 3rd

**0 093 266**

and 4th main group of Mendeleev's periodic table, with a water content of 10 to 20 % by weight, are used as the inert inorganic adsorbent.

6. The process as claimed in Claims 1 to 3, characterised in that monomethylation of digoxin is carried out in the presence of strontium hydroxide octahydrate without the adddition of an inert inorganic adsorbent.

**Revendications**

1. Procédé pour la préparation de β-méthyldigoxine par monométhylation sélective de digoxine avec du sulfate de diméthyle dans un mélange de diméthylformamide et de toluène à titre de solvant, en présence d'un composé de strontium basique et éventuellement d'un agent adsorbant inorganique inerte, ainsi que par purification du produit de la méthylation par chromatographie en colonne sur $SiO_2$ avec un mélange d'hydrocarbure chloré et d'un alcool aliphatique inférieur, caractérisé en ce qu'on utilise par mole de digoxine, à titre de composé de strontium basique, de 1 à 3 moles d'hydroxyde de strontium octahydraté et, comme agent adsorbant inorganique inerte éventuellement présent, de 1 à 3 moles d'un oxyde, silicate ou phosphate d'éléments des 2ème, 3ème, 4ème ou 5ème groupes principaux ou secondaires de la Classification Périodique des Eléments de Mendeléeff, avec une teneur en eau allant jusqu'à 20 % en poids, et en ce qu'on effectue la réaction à des températures allant de − 15 °C à 15 °C.

2. Procédé suivant la revendication 1, caractérisé en ce qu'on effectue la réaction à des températures de − 5 °C à 5 °C.

3. Procédé suivant les revendications 1 et 2, caractérisé en ce qu'on utilise, par mole de digoxine, 2 moles d'hydroxyde de strontium octahydraté.

4. Procédé suivant l'une quelconque des revendications 1 à 3, caractérisé en ce qu'on utilise, par mole de digoxine, 3 moles de l'agent adsorbant inorganique inerte.

5. Procédé suivant l'une quelconque des revendications 1 à 4, caractérisé en ce qu'on utilise, comme agent adsorbant inorganique interne, des oxydes des éléments des 2ème, 3ème et 4ème groupes principaux de la Classification Périodique des Eléments de Mendeléeff, avec une teneur en eau de 10 à 20 % en poids.

6. Procédé suivant l'une quelconque des revendications 1 à 3, caractérisé en ce que la monométhylation de digoxine est effectuée en présence d'hydroxyde de strontium octahydraté sans addition d'un agent adsorbant inorganique inerte.

6